# EUROPEAN PATENT SPECIFICATION

(11) **EP 0 462 376 B1**
(45) Date of publication and mention of the grant of the patent: **24.07.1996**
(21) Application number: 91106593.6
(22) Date of filing: 24.04.1991
(51) Int. Cl.: G01N 33/543, G01N 33/558, G01N 33/564, G01N 33/94

(54) **Conjugate recovery binding assays**
Bindungsnachweisverfahren mit Konjugatrückgewinnung
Essais faisant intervenir de liaisons avec récupération de conjugue

(30) Priority: 09.05.1990 US 521053
(43) Date of publication of application: 27.12.1991
(73) Proprietor: ABBOTT LABORATORIES, Abbott Park, Illinois 60064-3500 (US)
(72) Inventor: Ching, Shanfun, Libertyville, Illinois 60048 (US); Jou, Tsung-Hui K., Vernon Hills, Illinois 60061 (US); Gordon, Julian, Lake Bluff, Illinois 60044 (US)
(74) Representative: Modiano, Guido, Dr.-Ing.

(56) References cited:
- EP-A- 0 201 339
- EP-A- 0 253 464
- EP-A- 0 284 232
- EP-A- 0 299 428
- WO-A-91/05262

## Description

### BACKGROUND OF THE INVENTION

### 1. Field of the Invention

The present invention relates to methods for detecting the presence or amount of an analyte in a test sample by means of a binding assay. In particular, the invention relates to methods in which a dual readout system by instrumental means is used for the detection of conjugate at a capture site and/or a conjugate recovery site.

### 2. Related Art

Various analytical procedures and devices are commonly employed in assays to determine the presence and/or amount of substances of interest or clinical significance which may be present in biological or non-biological fluids. Such substances are commonly termed "analytes" and can include antibodies, antigens, drugs, hormones, etc.

The ability to use materials which specifically bind to an analyte of interest has created a burgeoning diagnostic device market based on the use of binding assays. Binding assays incorporate specific binding members, typified by antibody and antigen immunoreactants, wherein one member of the specific binding pair is labeled with a signal-producing compound (e.g., an antibody labeled with an enzyme, a fluorescent compound, a chemiluminescent compound, a radioactive isotope, a direct visual label, etc.). For example, in a binding assay the test sample suspected of containing analyte can be mixed with a labeled anti-analyte antibody, i.e., conjugate, and incubated for the immunoreaction to occur. The reaction mixture is subsequently analyzed to detect either the label associated with an antibody/analyte complex (bound conjugate) or the labeled antibody which is not complexed with analyte (free conjugate) such that the amount of label in one of the species can be correlated to the amount of analyte in the test sample.

The solid phase assay is a commonly used binding assay technique. There are a number of assay devices and procedures wherein the presence of an analyte is indicated by the analyte's binding to a conjugate and/or an immobilized complementary binding member that is bound to a solid phase such as a dipstick, test strip, flow-through pad, paper, fiber matrix or other solid phase material. The binding assay results in a distribution of the conjugate between that which is immobilized upon the solid phase and that which remains free. Typically, the presence or amount of analyte in a test sample is indicated by the extent to which the conjugate becomes immobilized upon the solid phase.

The use of reagent-impregnated test strips in specific binding assays is also well-known. In such procedures, a test sample is applied to one portion of the test strip and is allowed to migrate or wick through the strip material. Thus, the analyte to be detected or measured passes through the material, usually with the aid of an eluting solvent which can be the test sample itself or a separately added solvent. The analyte progresses into a detection zone on the test strip wherein a complementary binding member to which the analyte can bind is immobilized. The extent to which the analyte becomes bound in the detection zone can be determined with the aid of the conjugate which can also be incorporated in the test strip or which can be applied separately.

Examples of devices based upon these principles include the following patents and patent applications. A test strip device is described by Deutsch et al. in U.S. Patent Nos. 4,094,647, 4,235,601 and 4,361,537. In general, the device comprises a material capable of transporting a solution by capillary action, i.e., wicking. Different areas or zones in the strip contain the reagents needed to produce a detectable signal as the analyte is transported to or through such zones. The device is suited for both chemical assays and binding assays and uses a developer solution to transport analyte along the strip.

Tom et al. (U.S. Patent No. 4,366,241) disclose a bibulous support with an immunosorbing zone where the test sample is applied and the assay result is read. Weng et al. (U.S. Patent No. 4,740,468) disclose a test strip device wherein the binding of a labeled-specific binding pair member to an immobile second specific binding pair member, at a small situs on a bibulous strip, enables the observation of a detectable signal at the small situs, with the proviso that, if the immobile second specific binding pair member could directly bind the labeled-specific binding pair member then an analyte-analog capable of binding the labeled-specific binding pair member is also present on the strip to remove free or unbound conjugate before it reaches the small situs detection site. Greenquist (U.S. Patent No. 4,806,311) discloses a multizoned or multilayered device having a detection zone and a reagent zone which removes free monovalent labeled-binding members from the test medium.

Weng et al. (U.S. Patent No. 4,740,468) disclose a device for use with a test solution including sample, suspected of containing analyte, and a first specific binding member which binds to the analyte and which is labeled. The device is a teststrip having a second specific binding member, immobilized at a small detection situs, which binds to labeled analyte. The strip also includes an analyte analog to bind any first specific binding member which is capable of binding the second specific binding member in the absence of analyte.

Other test strip descriptions of interest are presented in U.S. Patent No. 4,861,711; EP 282232, published September 28, 1988; WO 88/08534, published November, 3, 1988; WO 88/08536, published November 3, 1988; and EP 286,371, published November 12, 1988. Also of interest is the Mickelson et al. disclosure of a method and test device for separating labeled reagent by binding free labeled reagent to microparticles which will not pass through a solid phase device (EP 297,292).

Of further interest is EP 201 339 which discloses a device comprising a reaction chamber having nonoverlapping first and second reaction surfaces, the first reaction surface having immobilized thereon an analyte binding partner having an analyte conjugate (a labeled analyte) reversibly bound thereto and the second reaction surface having immobilized thereon a binding partner for the analyte conjugate. Signal is detected at the second reaction surface.

There is increased manufacturer activity in the test strip field because there is a growing demand for devices that require few or no manipulative steps to perform the assay, that can be used by untrained personnel, and that provide results that are minimally affected by variations in the manner of assay performance. Further considerations are the ease with which the observed signal can be detected as well as the ease with which conjugate immobilized at the detection site can be distinguished from conjugate which passed through the detection site. In addition, a need exists for an assay format which converts a small decrease in detectable signal into a more readily detected appearance of signal and which enables the detection of both such signals. Such a format would also enhance the sensitivity of the assay, i.e., smaller amounts of analyte could be detected. Accordingly, there remains a need for new devices which are easy to use, accurate and rapid, and which incorporate self-performing assay controls.

### SUMMARY OF THE INVENTION

The present invention provides methods for determining the amount of an analyte in a test sample. The devices for carrying out the methods of the invention involve a solid phase material through which the analyte and a conjugate (e.g., a label attached to a binding member specific for the analyte) are capable of migrating. The solid phase includes at least two defined and marked detection sites, in sequential fluid-flow contact, for the immobilization and comparative display of the conjugate or complexes thereof. A first detection site involves a capture site having affixed thereto an immobilized capture reagent capable of competing with the analyte for binding to the conjugate. Alternatively, the capture site involves a capture reagent to which the analyte and the conjugate competitively bind. A second detection site involves a conjugate recovery site having affixed thereto a conjugate recovery reagent, that is different from the capture reagent, and that is capable of binding either the conjugate or a complex thereof. The conjugate recovery site receives and binds those analyte/conjugate complexes which pass through the capture site. As the amount of analyte in the test sample increases, the more the binding sites of the conjugate are occupied by analyte molecules and the less conjugate is free to bind the capture reagent. Instead, the analyte/conjugate complexes pass through the capture site and migrate into the conjugate recovery site where the complexes become immobilized by the conjugate recovery reagent. Thus, as the analyte concentration increases, the conjugate immobilized at the capture site decreases and the conjugate immobilized at the recovery site increases.

The present invention derives its principal advantages from the use of the conjugate and/or the use of the dual readout format. The conjugate can include a specific binding member conjugated to a detectable label wherein the binding member will simultaneously bind more than one analyte. Optionally, the conjugate can include more than one specific binding member attached to a detectable label. In either instance, substantially all of the binding sites on the conjugate are occupied by analyte before the conjugate will pass through the capture site without binding to the capture reagent.

The dual readout format uses a minimum of two defined sites for the comparative display of that conjugate which becomes immobilized within the sites; for example, a labeled-antibody conjugate having unoccupied binding sites after reacting with the analyte of the sample will become immobilized at the analyte-analog capture site, while conjugate having substantially no unoccupied binding sites after reacting with the sample will pass through the capture site and become immobilized at the conjugate recovery site where the immobilized reagent is capable of binding the label or the specific binding member of the conjugate, an epitope peculiar to the conjugate, the analyte or an ancillary specific binding member. No unreactive migratable species is produced and the use of two binding sites for a comparative display of the different reaction complexes results in a highly sensitive binding assay which is non-obvious over the prior art.

In an alternative embodiment, the conjugate includes a label attached to a specific binding member having a binding site specific for the analyte as well as a binding site specific for the capture reagent, wherein the binding of the analyte to the conjugate inhibits the binding of the capture reagent to the conjugate, and wherein the binding of the conjugate to the capture reagent inhibits the binding of the analyte to the conjugate. For example, the conjugate can be a label attached to an antibody, wherein the analyte is specific for a constant region of the antibody and the capture reagent is specific for a variable region of the antibody, and wherein both the analyte and the capture reagent are inhibited from simultaneously binding to the conjugate. The solid phase is incorporated with at least two defined and optionally marked detection sites, in sequential fluid-flow contact, for the immobilization and comparative display of the conjugate or a complex thereof, and the conjugate recovery site comprises a capture reagent that is capable of binding the analyte/conjugate complexes that pass through the capture site.

In addition, the devices disclosed herein can involve flow-through pads or layered devices as well as test strip devices made of one or more different materials. Modifications of the devices involve the inclusion of test sample application pads or layers, filters, flow control layers, blocking layers and absorbent pads. The conjugate can be contained on or in the device at a position upstream from the capture site, it can be reversibly bound to the capture reagent or it can be contacted to the device individually or as a test solution when mixed with the test sample. Similarly, any additional signal producing components that are needed to generate a detectable signal can be contained on or in the device or be individually added.

The assay methods involve contacting a test sample suspected of containing the analyte to a solid phase device, as described above, and allowing the test sample to migrate through the solid phase and sequentially contact the capture site and the conjugate recovery site. Detectable complexes are formed from the analyte, the conjugate, the capture reagent and the conjugate recovery reagent, and optionally from ancillary specific binding members. These complexes are then detected at both the capture site and the conjugate recovery site, by instrumental means. The comparative signals from the sites are used to determine the amount of analyte in the test sample. In methods wherein the conjugate is previously complexed to the capture reagent, the analyte present in the test sample displaces the conjugate which in turn is recaptured by the conjugate recovery reagent at the conjugate recovery site.

The advantageous use of the conjugate and comparative binding sites to detect the amount of analyte in the test sample provides a wide dynamic assay range; small amounts of analyte are more readily detected, and samples containing large amounts of analyte can be assayed without the need for additional dilution steps. In addition, the present invention can be used in a multianalyte assay method and provides assay procedure controls to detect the adulteration of a test sample.

### DETAILED DESCRIPTION OF THE INVENTION

As will be described in greater detail hereinafter, the assay devices used to carry out the method of the present invention involve at least one capture site and at least one conjugate recovery site at which detectable signals are produced. The capture and conjugate recovery sites are each incorporated with an immobilized reagent which is not capable of being insolubilized or otherwise removed from the site. In general, the capture reagent in the capture site competes with the analyte of the test sample for binding to a conjugate, i.e., a labeled-specific binding member. Preferably, the conjugate provides multiple sites for binding to the analyte. When substantially all of the binding sites of the conjugate are occupied by analyte, the analyte/conjugate complex is free to pass through the capture site without the conjugate binding to the capture reagent and without the analyte/conjugate complex being immobilized by the capture reagent. The reagent of the conjugate recovery site will then bind and immobilize any analyte/conjugate complex which has passed through the capture site. A comparative analysis or measurement of the amounts of label at each site indicates the amount of analyte in the test sample. The two sites also provide advantageous assay control functions which will be described in detail hereinafter.

Before proceeding with the description of the specific embodiments of the present invention, a number of terms used herein will be defined.

"Test sample" refers to a material suspected of containing the analyte and used directly as obtained from the source or after pretreatment so as to modify its character. The test sample can be derived from any source, such as a physiological fluid, including, blood, saliva, ocular lens fluid, cerebral spinal fluid, sweat, urine, milk, ascites fluid, mucous, synovial fluid, peritoneal fluid, amniotic fluid or the like. The fluid can be pretreated prior to use, such as preparing plasma from blood, diluting viscous fluids, or the like; methods of treatment can involve filtration, distillation, concentration, inactivation of interfering components, and the addition of reagents. Besides physiological fluids, other liquid samples can be used such as water, food products and the like for the performance of environmental or food production assays as well as diagnostic assays. In addition, a solid material suspected of containing the analyte can be used as the test sample once it is modified to form a liquid medium or to release the analyte.

"Specific binding member" refers to a member of a specific binding pair, i.e., two different molecules wherein one of the molecules specifically binds to the second molecule through chemical or physical means. In addition to antigen and antibody specific binding pair members, other specific binding pairs include, as examples without limitation, biotin and avidin, carbohydrates and lectins, complementary nucleotide sequences, complementary peptide sequences, effector and receptor molecules, enzyme cofactors and enzymes, enzyme inhibitors and enzymes, a peptide sequence and an antibody specific for the sequence or the entire protein, polymeric acids and bases, dyes and protein binders, peptides and specific protein binders (e.g., ribonuclease, S-peptide and ribonuclease S-protein), and the like. Furthermore, specific binding pairs can include members that are analogs of the original specific binding member, for example an analyte-analog or a specific binding member made by recombinant techniques. If the specific binding member is an immunoreactant it can be, for example, an antibody, antigen, hapten, or complex thereof, and if an antibody is used, it can be a monoclonal or polyclonal antibody, a recombinant protein or antibody, a mixture(s) or fragment(s) thereof, as well as a mixture of an antibody and other specific binding members. The details of the preparation of such antibodies and their suitability for use as specific binding members are well-known to those skilled-in-the-art.

"Analyte" or "analyte of interest" refers to the compound or composition to be detected or measured, which has at least one epitope or binding site. The analyte can be any substance for which there exists a naturally occurring analyte-specific binding member or for which an analyte-specific binding member can be prepared. Analytes include, but are not limited to toxins, organic compounds, proteins, peptides, microorganisms, amino acids, nucleic acids, hormones, steroids, vitamins, drugs (including those administered for therapeutic purposes as well as those administered for illicit purposes), and metabolites of or antibodies to any of the above substances. The term "analyte" also includes any antigenic substances, haptens, antibodies, macromolecules and combinations thereof.

"Analyte-analog" refers to a substance which cross-reacts with the analyte-specific binding member, although it may do so to a greater or a lesser extent than does the analyte itself. The analyte-analog can include a modified analyte as well as a fragmented or synthetic portion of the analyte molecule, so long as the analyte-analog has at least one epitopic site in common with the analyte of interest. An example of an analyte-analog is a synthetic peptide sequence which duplicates at least one epitope of the whole-molecule analyte so that the analyte-analog can bind to the analyte-specific binding member.

"Conjugate" refers to a substance comprising a detectable label covalently or noncovalently attached to a specific binding member. The method of attachment is not critical to the present invention. The label enables the conjugate to produce a detectable signal that is directly or indirectly related to the amount of analyte in the test sample. The specific binding member component of the conjugate is selected to directly bind to the analyte or to indirectly bind the analyte by means of an ancillary specific binding member, which is described in greater detail hereinafter. The conjugate can be incorporated into the test strip or device at a site upstream from the capture site, it can be combined with the test sample to form a test solution, it can be added to the test strip or device separately from the test sample or it can be predeposited or reversibly immobilized at the capture site.

"Label" refers to any substance which is capable of producing a signal that is detectable by instrumental means. Various labels suitable for use in the present invention include labels which can produce signals through either chemical or physical means and can include enzymes and substrates; chromogens; catalysts; fluorescent compounds; chemiluminescent compounds; radioactive labels; direct visual labels including colloidal metallic particles such as gold, colloidal non-metallic particles such as selenium, dyed or colored particles such as a dyed plastic or a stained microorganism, organic polymer latex particles and liposomes or other vesicles containing directly visible substances; and the like.

The selection of a particular label is not critical, but the label will be capable of generating a detectable signal either by itself, such as a visually detectable colored organic polymer latex particle or an instrumentally detectable fluorescent compound, or in conjunction with one or more additional signal producing components, such as an enzyme/substrate signal producing system. A variety of different conjugates can be formed by varying either the label or the specific binding member; it will be appreciated by one skilled-in-the-art that the choice involves consideration of the analyte to be detected and the desired means of detection.

"Signal producing component" refers to any substance capable of reacting with another assay reagent or with the analyte to produce a reaction product or signal that indicates the presence of the analyte and that is detectable by instrumental means. "Signal production system", as used herein, refers to the group of assay reagents that are needed to produce the desired reaction product or signal. For example, one or more signal producing components can be reacted with the label to generate a detectable signal, e.g., when the label is an enzyme, amplification of the detectable signal is obtained by reacting the enzyme with one or more substrates or additional enzymes to produce a detectable reaction product.

A large number of enzymes suitable for use as labels are disclosed in U.S. Patent No. 4,275,149, columns 19-23. An example of an enzyme/substrate signal producing system useful in the present invention is the enzyme alkaline phosphatase, wherein the substrate used is nitro blue tetrazolium-5-bromo-4-chloro-3-indolyl phosphate or a derivative or analog thereof.

In an alternative signal producing system, the label can be a fluorescent compound where no enzymatic manipulation of the label is required to produce the detectable signal. Fluorescent molecules such as fluorescein, phycobiliprotein, rhodamine and their derivatives and analogs are suitable for use as labels in such a system.

In a preferred embodiment of the present invention, a visually detectable particle is used as the label component of the conjugate, thereby providing for a instrumental readout of the concentration of the analyte in the sample without the need for additional signal producing components at the reactive readout sites. Materials for use as the particle/labels are colloidal metals, such as gold, and dye particles as disclosed in U.S. Patent Nos. 4,313,734 and 4,373,932.
The preparation and use of non-metallic colloids, such as colloidal selenium particles, are disclosed in co-owned EP 298 368, published January 11, 1989.
The use of colloidal particle labels with test strips is disclosed in co-owned EP 299 428, published January 18, 1989. Organic polymer latex
particles for use as labels are disclosed in co-owned EP 360 088, published March 28, 1990.

"Solid phase" refers to any suitable chromatographic, bibulous, porous, isotropic or capillary material which forms the basis of the test device. The assay device disclosed herein can have many configurations, several of which are dependent upon the material chosen for the solid phase. For example, the assay device can include a solid phase material configured for use in a flow-through assay device, a chromatographic column, a dipstick or a test strip. It will be appreciated by one skilled-in-the-art that a test strip device can be made of more than one material (e.g., different zones or sites can be made of different materials) and a flow-through device can have more than one layer, wherein different layers can be made of different materials, so long as the multiple materials or layers are in fluid-flow contact with one another thereby enabling the passage of test sample between the materials or layers. Fluid-flow contact permits the passage of at least some components of the test sample, e.g., analyte, between the zones or layers of the device and is preferably uniform along the contact interface between the fluid-flow zones or layers. In a flow-through device disclosed herein a blocking layer may be present between the capture site and conjugate recovery site such that the presence or amount of label immobilized at each site can be separately detected and/or measured without an interfering signal from the other site. It will also be appreciated that those zones or layers of the device which contain an assay reagent can be separated by zones or layers which do not contain reagents so long as each of the zones, layers or reagent sites are in continuous fluid-flow contact; this is referred to as the indirect fluid-flow contact of reagent containing zones or layers. To simplify the disclosure hereinafter, the test device will be described principally as comprising a test strip structure containing at least the immobilized binding reagents necessary for the performance of the dual readout system.

"Capture reagent" refers to a specific binding member that is affixed or non-diffusively attached within or upon a portion of the solid phase to form a "capture site". The method of attachment is not critical to the present invention. The capture reagent is selected to bind the conjugate or a complex thereof. In geheral, the capture reagent competes with the analyte for binding to the conjugate. Alternatively, the analyte and labeled reagent (e.g., labeled analyte) can compete for binding to the capture reagent (e.g., analyte-specific antibody). The capture reagent can be chosen to directly bind the conjugate or indirectly bind the conjugate by binding to an ancillary specific binding member which is bound to the conjugate.

The capture site is a delimited or defined portion of the solid phase such that the specific binding reaction of the capture reagent and analyte or conjugate is localized or concentrated to facilitate the detection of label immobilized at the capture site in contrast to other portions of the solid phase. The capture reagent can be applied to the solid phase by dipping, inscribing with a pen, dispensing through a capillary tube or through the use of reagent jet-printing as described in the co-owned EP 268 237, published May 25, 1988.

In addition, the capture site can be marked, for example with a dye, such that the position of the capture site upon the solid phase can be visually or instrumentally determined even when there is no label immobilized at the site. The capture reagent is present on the device in an amount sufficient to bind or retain substantially all of the conjugate in the absence of analyte.

"Conjugate recovery reagent" refers to a specific binding member that is affixed within or upon a portion of the test strip, downstream from the capture site, to form a "conjugate recovery site", i.e., the conjugate recovery site is that region of the solid phase where conjugate that has not been immobilized at the capture site is recovered from the migrating fluid or test sample. The conjugate recovery site is in direct or indirect fluid-flow contact with the capture site such that the test sample is allowed to diffuse or permeate into and through the capture site and subsequently pass into the conjugate recovery site. The method of attaching the conjugate recovery reagent to the solid phase is not critical to the present invention.

The conjugate recovery reagent is selected to bind, and thereby immobilize, the conjugate or complexes thereof. For example, the conjugate recovery reagent can be chosen to bind the label portion of the conjugate, the specific binding member portion of the conjugate or an epitope formed by the conjugation of the label and specific binding member, as well as to a conjugate/ancillary specific binding member complex or a conjugate/analyte complex. Similar to the capture site, the conjugate recovery site of the present invention is a delimited or defined portion of the solid phase such that the specific binding reaction is localized or concentrated to facilitate the detection of label immobilized at the site in contrast to other portions of the solid phase. The conjugate recovery site can be marked such that the position of the site upon the solid phase can be visually or instrumentally determined even though there is no label immobilized at the site. The conjugate recovery reagent can be applied to the solid phase by techniques similar to those described above for the capture reagent. Preferably, the conjugate recovery reagent is present on the device in an amount sufficient to bind or retain substantially all of the conjugate which is displaced from or which passes through the capture site, i.e., the amount is generally at least equal to the amount of analyte normally expected to be present in a test sample. Alternatively, the conjugate recovery reagent immobilized at the recovery site can be present in an amount which exceeds the amount of analyte expected to be present in the sample. Alternative methods can be used to perform the separation step, such as affixing to the solid phase a binding member that is specific for mobile conjugate recovery reagent; or affixing to the solid phase a reactive agent, such as a charged substance, which will attract and bind an oppositely charged substance that has been bound to a mobile conjugate recovery reagent, as disclosed in co-owned EP 326 100, published August 2, 1989.

"Ancillary specific binding member" refers to any member of a specific binding pair which is used in the assay in addition to the specific binding members of the conjugate, capture reagent or conjugate recovery reagent. One or more ancillary specific binding members can be used in an assay. For example, an ancillary specific binding member can be capable of binding the conjugate to the analyte of interest, in instances where the analyte itself could not directly attach to the conjugate, or the ancillary specific binding member can be used to increase the amount of conjugate which can complex with the analyte in a binding reaction. The ancillary specific binding member can be incorporated into the assay device or it can be added to the device as a separate reagent solution.

"Analyte derivative" refers to any substance whose concentration in the test sample or assay reaction mixture is directly proportional to the analyte concentration. For example, the derivative may be a complex of the analyte and an ancillary specific binding member which in turn binds to an affixed member. As another example, the derivative can be a reaction product formed in stochiometric relationship to the analyte concentration, wherein the reaction product binds to an affixed member. Thus, an analyte derivative is any substance quantitatively related to analyte concentration.

### Assay Methods

One method of the invention is performed by first combining the test sample and a conjugate, such as a labeled analyte-specific binding member thereby forming a test solution. The test solution is contacted to a solid phase device, such as a test strip. The strip includes: an application site to which the test solution is contacted; a capture site to which is affixed a capture reagent (e.g., an analyte-analog) that is capable of competing with the analyte for binding to the conjugate; and a conjugate recovery site, downstream from the capture site, to which is affixed a conjugate recovery reagent capable of immobilizing any conjugate which passed through the capture site. The detectable signals at both the capture site and the conjugate recovery site are observed to determine the assay result. In this assay, the more analyte present in the test sample, the less conjugate is immobilized at the capture site and the more conjugate is immobilized at the conjugate recovery site. The dual detection sites allow both results to be observed and quantitated. The comparison of the result at the capture site to that at the conjugate recovery site provides increased assay sensitivity because relatively small decreases in signal at the capture site will appear as relatively large increases of signal at the conjugate recovery site, where there previously was no signal. In addition, when starting with a known amount of conjugate, the proportions of conjugate immobilized at the two detection sites can be compared to provide a ratio which can be used to quantitatively or semi-quantitatively assess the amount of analyte present in the test sample.

In a preferred embodiment of the present invention, a polyvalent conjugate is used. The polyvalent conjugate is capable of simultaneously binding to more than one analyte molecule. For example, the specific binding member component of the conjugate can be polyvalent, such as an antibody having multiple binding sites capable of binding multiple analyte molecules. Alternatively, the label can be conjugated to more than one monovalent or polyvalent specific binding member, e.g., a directly visible colloidal particle to which is bound multiple monovalent antibodies each of which will bind an analyte molecule. Substantially all of the analyte binding sites on the polyvalent conjugate must be occupied by an analyte molecule from the test sample, or the conjugate will become immobilized at the capture site where, for example, the conjugate binds to an analyte-analog capture reagent. Therefore, with the polyvalent conjugate of the present invention, a predetermined threshold amount of analyte must be present in the test sample before any of the conjugate will migrate through the capture site and pass to the conjugate recovery site.

The use of a polyvalent conjugate also supports the widening of the dynamic range of the assay method. A preferred polyvalent conjugate involves a colloidal particle label to which is attached a number of specific binding members. With this polyvalent conjugate, the assay's dynamic range will be determined by the number of specific binding members bound to the colloidal particle. Such a conjugate can be used to modulate the assay's dynamic range by controlling both the size of the colloidal particle, to accommodate more specific binding member, and the number of specific binding members per colloidal panicle. When there are many binding sites on the conjugate for the analyte to saturate, then proportionately more analyte is needed to displace the conjugate from the capture site or to prevent the conjugate from binding to the capture site, and there is an increased range over which there is competition between the analyte and an analyte-analog for the conjugate.

In modifications of the embodiments of the present invention, the test sample and conjugate can be individually contacted to the solid phase, or they can be combined to form a test solution which is contacted to the solid phase. In most preferred embodiments of the present invention, the conjugate is incorporated into the solid phase test device such that the assay is substantially self-performing once the test sample is contacted. The application site of the solid phase can be a portion of the device on which there is no reagent present, or the application site can be the capture site or a site at which mobile conjugate is present. In methods using a label which is not detectable by itself, the solid phase is contacted to any remaining members of a signal producing system that were not included with the test solution or were not present on the solid phase. Alternatively, the capture and conjugate recovery sites can be individually contacted to any remaining signal producing components.

Each of the embodiments of the invention can also include the use of ancillary specific binding members. One or more ancillary specific binding members can be placed at appropriate locations in or on the device, or be added separately thereto, to complete the binding of analyte, conjugate or analyte/conjugate complex at the capture site and/or conjugate recovery site. The ancillary specific binding members can include complexes formed from two or more specific binding members. In addition, ancillary binding members can be used to indirectly increase the number of binding sites on an analyte such that a greater amount of conjugate will bind thereto. For example, an antibody/biotin complex (i.e., an ancillary specific binding member comprising an antibody bound to multiple biotin molecules) can be used to bind a monovalent analyte (e.g., an antigen having a single binding site) to a plurality of conjugate molecules comprising labeled anti-biotin antibodies.

Yet another embodiment of the present invention uses a conjugate displacement/recapture format. For example, the capture site can contain an analyte-analog capture reagent to which a conjugate has been reversibly complexed. If there is no analyte in the test sample, then there is a detectable signal only at the capture site because no analyte was present to competitively bind with the conjugate and thereby displace the conjugate from the capture site. If analyte is present in the test sample, however, then the conjugate is displaced from the capture site, binds to the analyte and subsequently passes to the conjugate recovery site which contains a specific binding member capable of directly or indirectly binding to the conjugate or a complex thereof. Thus, the greater the amount of analyte present in the test sample, the greater the amount of conjugate displaced from the capture site, and the greater the detectable signal at the conjugate recovery site relative to the detectable signal at the capture site. The use of the predeposited conjugate, reversibly bound at the capture site, provides assurance that the decrease of signal at the capture site is not simply a consequence of a non-specific inhibition of conjugate capture at the capture site. In examples of alternative displacement/recapture formats the capture site can contain an analyte-specific binding member capture reagent to which the conjugate, e.g., labeled analyte-analog, has been previously complexed, or the capture site can contain an analyte or analyte-analog to which the conjugate, e.g., a labeled analyte-specific binding member, has been reversibly complexed.

A particularly preferred method of the present invention uses the displacement/recapture format together with a conjugate comprising a direct visual label attached to a specific binding member. The use of the direct visual labels enables the detection of signal changes at both the capture and conjugate recovery sites without the need to add additional signal producing reagents.

In yet another embodiment of the present invention, the conjugate comprises a label attached to a specific binding member, wherein the binding member has binding sites specific for the analyte as well as separate binding sites specific for the capture reagent. The capture reagent can be selected to bind a site on the specific binding member portion of the conjugate which is different from the analyte-binding site on the specific binding member portion of the conjugate, and the binding of the capture reagent prevents or inhibits the binding of analyte to the conjugate. Alternatively, the binding of the analyte prevents or inhibits the binding of the capture reagent to the conjugate. The capture reagent is neither an analyte, analyte-analog or analyte-derivative in this embodiment. One explanation of this competitive binding competition is steric hindrance. For example in an assay for rheumatoid factor (IgM), the conjugate can be a labeled anti-biotin antibody (labeled-lgG) and the capture reagent can be biotin, wherein the analyte and capture reagent can compete for binding to the antibody component of the conjugate. The conjugate antibody has a constant region or domain (isotype fragment) to which certain specific binding members bind and at least one variable region or domain (idiotype fragment) to which different specific binding members bind. In this example, the IgM analyte, which is an anti-lgG antibody, binds to an isotypic epitope region of the lgG molecule, while the biotin capture reagent binds to the idiotypic epitope of the IgG molecule. The binding of the analyte to the conjugate affects the lgG molecule such that the molecule is unable to simultaneously bind the capture reagent. The exact cause of such binding interference is not critical to the present invention; one skilled-in-the-art can empirically determine whether the binding of an analyte to a conjugate inhibits the conjugate's subsequent binding to a capture reagent.

This embodiment of the present invention also enables the performance of a combination competitive/sandwich assay on the dual readout devices. In the rheumatoid factor example, the conjugate recovery reagent can be an analyte-specific binding member which directly binds the analyte portion of the analyte/conjugate complex which passes through the capture site, thereby forming a capture reagent/analyte/conjugate sandwich.

The conjugates and dual readout format are also advantageous in the performance of assays to detect the amount of more than one analyte in the test sample. For example in a multi-drug screening assay method and device, a capture site can be present for each different analyte to be detected; one capture site can contain a cocaine analyte-analog, a second can contain an opiate analyte-analog, and a third can contain an amphetamine analyte-analog. A conjugate, comprising a label and a suitable analyte-specific binding member, can be reversibly complexed to the analyte-analog in each capture site. The conjugate recovery site can comprise a specific binding member which will immobilize any of the conjugate/analyte complexes which are formed in the presence of analyte and which pass through the capture sites and migrate to the recovery site. The dual readout system of the present invention will indicate the presence of a drug or drugs via the decrease in label at one or more capture sites and the increase in label at the conjugate recovery site. A modification of the multianalyte assay can include the use of multiple conjugate recovery sites. If it is necessary to know which drug is present in the test sample, then a different conjugate recovery reagent can be used in separate conjugate recovery sites for each analyte such that a given analyte can be detected by both a decreasing and an increasing signal. Alternatively if it is only necessary to know if any one of the drugs is present, then a single conjugate recovery reagent (a "generic" conjugate recovery reagent in which the specific binding member component will bind to any of the conjugates used) can be used in multiple sites to form a ladder-type configuration, which is described in further detail hereinafter; the greater the number of recovery sites at which the label is detectable, the greater the amount of an analyte or a combined amount of analytes in the test sample.

The novel methods of the present invention increase the assay's reliability and sensitivity by enabling the detection and/or measurement of decreasing and increasing signals at two different sites for a single analyte. The detection of assay results at the two sites is performed by an instrument reader. The observation of the signal at both the capture site and the conjugate recovery site provides a double-check on the validity of the assay result.

By using the procedure with appropriately titrated quantities of conjugate, capture reagent and conjugate recovery reagent, an extended range of accuracy can be obtained for a quantitative or semi-quantitative assay. When using the conjugate displacement/recapture format at low analyte concentrations, there is only a small decrease in signal at the capture site, but there is greater sensitivity at the conjugate recovery site where the appearance of even a low signal is more readily observed. At high analyte concentrations, there is greater sensitivity at the capture site because the decrease in signal is large, and the increasing signal at the conjugate recovery site is readily detected and serves to validate quantitative results. It will be appreciated by those skilled-in-the-art that, with the appropriate choice of reagent concentrations, the device can be made such that the two detection sites have non-overlapping optimal sensitivities.

Another favorable aspect of the dual readout system is the ability to detect the adulteration of test samples, an advantageous assay control which is especially important in the abused drugs field. In conventional immunoassay methods, if a urine test sample is adulterated with a substance such as bleach, household detergent or an acid or a base, then the immunoreaction is inhibited, and a false negative result is observed at the detection site in a sandwich assay format; the adulterant inhibits the binding reaction, and the inhibition is manifested as an assay result indicating the absence of analyte. With the dual readout system, however, adulteration of the test sample will result in the absence of signal at both the capture site and the conjugate recovery site, thereby making the adulteration immediately apparent.

Yet another assay control aspect of the invention is provided by the dual readout format in comparison to the single detection sites of prior art devices. In prior devices, the capture site will not display a detectable signal if the test sample contains an abnormally high amount of analyte, e.g., the analyte overloads the available conjugate binding sites so that no conjugate is able to bind at the capture site. In the present invention, however, conjugate having binding sites totally occupied by analyte will still be immobilized at the conjugate recovery site. Therefore, even if the test sample contains an abnormally high amount of analyte, the devices disclosed herein will detect the presence or amount of that analyte.

### Assay Device

The assay device disclosed herein can be any suitably absorbent, porous, isotropic or capillary possessing material through which a solution or fluid containing the analyte can travel. The solution can be pulled or pushed through the solid phase by suction, hydraulic, pneumatic, hygroscopic or capillary forces, or by a combination thereof. Possible assay devices include, but are not limited to, a conventional chromatographic column, an elongated strip of material wherein the fluid-flow is substantially linear, and a sheet wherein the fluid-flow is linear or radial. As described above, if a flow-through device is made, a blocking layer is generally present between the capture site and conjugate recovery site such that the presence or amount of label immobilized at each site can be separately detected and/or measured without the occurrence of an interfering signal. For example, a suitable blocking layer component for use with chromogenic or colloidal particle labels can include any opacifying material which will inhibit the signal of one site from being detectable at the other readout site or sites.

Natural, synthetic, or naturally occurring solid phase materials that are synthetically modified, can be used and include: papers (fibrous) or membranes (microporous) of cellulose materials such as paper, cellulose, and cellulose derivatives such as cellulose acetate and nitrocellulose; fiberglass; cloth, both naturally occurring (e.g., cotton) and synthetic (e.g., nylon); porous gels such as silica gel, agarose, dextran, and gelatin; porous fibrous matrixes; starch based materials, such as Sephadex® brand cross-linked dextran chains; ceramic materials; olefin or thermoplastic materials including films of polyvinyl chloride, polyethylene, polyvinyl acetate, polyamide, polycarbonate, polystyrene, copolymers of vinyl acetate and vinyl chloride and combinations of polyvinyl chloride-silica; and the like. The solid phase material should not interfere with the production of a detectable signal. If a strip or sheet is used, then the material should have a reasonable inherent strength, or strength can be provided by means of a supplemental support.

A preferred solid phase material is nitrocellulose. Especially when membranous solid phase material is used, the test sample and conjugate may be mixed prior to initiating fluid-flow through the solid phase to obtain a controlled, reproducible binding reaction between the analyte and the conjugate. Alternatively, the test device can further include a premixing application pad containing the conjugate. Such a device is disclosed in co-owned EP 323 605, published July 12, 1989.

The material of the application pad should be chosen for its ability to premix the test sample with the conjugate. If nitrocellulose is used as the solid phase, then Porex® hydrophilic polyethylene frit or glass fiber filter paper are appropriate application pad materials. Alternatively, if a solid phase material such as glass fiber filter paper is used, then the conjugate can be reversibly immobilized on the strip either at the sample application site or at another site upstream of the capture site. In yet other alternative devices and methods, the conjugate can be added to the device as a separate reagent solution, either sequentially or simultaneously with the test sample.

An application pad can be made of any material from which the test sample can pass to the test strip. Materials preferred for use in the application pad include porous polyethylene frit or pads and glass fiber pads or filter paper. The material must also be chosen for its compatibility with the analyte and assay reagents.

In addition, the application pad can contain one or more assay reagents either diffusively or non-diffusively attached thereto. Reagents which can be contained in the application pad include, but are not limited to, the conjugate, ancillary specific binding members, test sample pretreatment reagents and signal producing components. The isolation of assay reagents in the application pad also keeps interactive reagents separate and facilitates the manufacturing process.

The particular dimensions of the solid phase will be a matter of convenience and will depend upon the size of the test sample involved, the assay protocol, the means for detecting and measuring the signal, and the like. For example, the dimensions may be chosen to regulate the rate of fluid migration as well as the amount of test sample to be imbibed by the solid phase.

The capture site and/or the conjugate recovery site can be formed by directly or indirectly attaching an appropriate specific binding member to the test strip. Direct attachment methods include adsorption or covalent binding such as by use of (i) a cyanogen halide, e.g., cyanogen bromide or (ii) glutaraldehyde. If the test strip is made of nitrocellulose, it is preferable to directly immobilize the desired reagent on the test strip by adsorption.

It is not necessary that the capture reagent and conjugate recovery reagent be bound directly to the solid phase. The specific binding member can be attached to another material wherein that material is physically entrapped or retained and immobilized within the solid phase by a physical, chemical or biochemical means. For example, the specific binding member can be attached to insoluble microparticles which are in turn affixed to the solid phase. The means of attaching a reagent to the microparticles encompasses both covalent and non-covalent means. It is generally preferred that the capture reagent and conjugate recovery reagent be attached to the microparticles by covalent means. By "retained and immobilized" is meant that the particles, once on the test strip, are not capable of substantial movement to positions elsewhere within the material. The particles can be selected by one skilled-in-the-art from any suitable type of particulate material including materials composed of polystyrene, polymethylacrylate, polyacrylamide, polypropylene, latex, polytetrafluoroethylene, polyacrylonitrile, polycarbonate, glass or similar materials. The size of the particles is not critical, although generally it is preferred that the average diameter of the particles be smaller than the average pore or capillary size of the solid phase.

The size of the particles may vary depending upon the type of solid phase material used as well as the type of material from which the particle is made. For example, in a glass fiber assay device, glass and polystyrene particles should be of sufficient size to become entrapped or immobilized in the pores of the solid phase material and not move when confronted by the migrating fluid. In the same glass fiber matrix, much smaller latex particles can be used because the latex particles unexpectedly affix themselves to the glass fibers by an unknown mechanism; see co-owned EP 389 003, published September 29, 1990. Thus, unlike the pore size dependent glass and plastic particles, the latex particles are pore sized independent so that lot-to-lot variations in pore size of the solid phase will not adversely affect the performance of the device.

Predetermined amounts of signal producing components and ancillary specific binding members can be incorporated within the device, thereby avoiding the need for additional protocol steps or reagent additions. Thus, it is within the scope of this invention to attach more than one reagent to the microparticles to be immobilized within the test strip. For example, to slow or prevent the diffusion of the detectable reaction product in an enzyme/substrate signal producing system, the substrate can be immobilized within the test strip. The substrate can be immobilized by direct attachment to the test strip by methods well-known in the art, or the substrate may be immobilized by being covalently bound to insoluble microparticles which have been deposited in and/or on the test strip.

The capture reagent and the conjugate recovery reagent can be deposited singly or in various combinations on or in the test strip in a variety of configurations to produce different detection or measurement formats. For example, the capture reagent and conjugate recovery reagent can be deposited to form discrete binding sites which are substantially smaller than the area of the entire test strip.

Alternatively, the reagents can be distributed over large portions of the test strip in a substantially uniform manner to form the capture and conjugate recovery sites. The extent of signal production along the length of the capture and conjugate recovery sites is related to the amount of analyte in the test sample. The amount of analyte can be determined by a comparison of the length or distance of the resulting signals to those observed for calibrated standards of analyte or other calibration scale provided with the device by the manufacturer.

Alternative embodiments include, but are not limited to: distributing the reagents in a gradient pattern, i.e., a lesser amount of reagent at the downstream end than at the upstream end of either or both of the capture and conjugate recovery sites; distributing the appropriate assay and control reagents to form patterns including numerals, letters, dots and symbols such as "+/-", "%" or the like; or distributing the reagents as a series of parallel bars which are spaced from about the proximal end, i.e., the sample application end, of the strip to about the distal end, thereby creating a ladder-like capture site and/or conjugate recovery site configuration; or distributing the reagents as sequential rings surrounding a central test sample or test solution application site on a sheet-like device. Such reagent distributions are disclosed in co-owned EP 299 428, published January 18, 1989, and EP 389 003, published September 29, 1990.

The various signal display formats or patterns described above can also incorporate assay controls to confirm the efficacy of the assay reagents or device, the completion of the assay or the proper sequence of assay steps. It is also within the scope of this invention to have a reagent, at the distal end of the test strip, which indicates the completion of a binding assay (i.e., an end of assay indicator). For example, the end of the assay may be shown by the indicator's changing color upon contact with the test solution, wicking solution or a signal producing component. Reagents which would change color upon contact with an aqueous test solution include the dehydrated transition metal salts, such as CUSO₄, Co(NO₃)₂, and the like. The pH indicator dyes can also be selected to respond to the pH of the buffered wicking solution. For example, phenolphthalein changes from clear to intense pink upon contact with a wicking solution having a pH range between 8.0-10.0.

A test sample can be contacted to the test strip by applying the test sample to an application site or by immersing the application site in the test sample. In a sheet-like device having radial capture and conjugate recovery sites, the sample is applied to a central application site. In a test strip or column, the sample can be applied to any portion of the strip which is upstream of the capture site, or the application site can be the capture site. Prior to contacting the sample to the solid phase, the sample can also be mixed with additional reagents such as the conjugate, buffers or wicking reagents (i.e., reagents which facilitate the travel of the test sample through the solid phase). In a further embodiment, the test sample can be applied to one portion of the test strip, upstream of the capture site, with one or more of the additional reagents being applied to yet another portion of the test strip upstream of the test sample application site.

The device can be further modified by the addition of a filtration means. The filtration means can be a separate material placed above the application site or application pad, or placed between the application pad and the test strip. Alternatively, the material of the application site or pad can be chosen for its filtration capabilities. The filtration means can include any filter or trapping device used to remove particles above a certain size from the test sample. For example, the filter means can be used to remove red blood cells from a sample of whole blood, such that plasma is the fluid transferred to the test strip. Such filter means are disclosed by U.S. Patent No. 4,477,575 and WO Application No. 86/02192, published April 23, 1987. Optionally, the filter means can include a reagent or reagents to remove particles or interferents from the test sample.

Another embodiment of the device involves the use of an additional zone, layer or layers of material placed between the application site or pad and the remaining test strip to control the rate of flow of the test sample from the application site or pad to the test strip. Such flow regulation is preferred when an extended incubation period is desired for the reaction of the test sample and the reagent(s) at the application site or pad. Alternatively, such a zone or layer can contain an additional assay reagent(s) which is preferably isolated from the application site or pad reagents until the test sample is added, or it can serve to prevent unreacted sample or assay reagents from passing to the test strip.

In yet another embodiment, the device can include an absorbent material downstream from the conjugate recovery site. It will be appreciated that the absorbent material can serve to increase the amount of test sample and conjugate which passes through the capture and conjugate recovery sites on the solid phase.

When small quantities of non-aqueous or viscous test samples are applied to the device, it may be necessary to employ a wicking solution, preferably a buffered wicking solution, to facilitate the travel of the reagent(s) and test sample through the device. When an aqueous test sample is used, a wicking solution generally is not necessary but can be used to improve flow characteristics or adjust the pH of the test sample. In immunoassays, the wicking solution typically has a pH range from about 5.5 to about 10.5, and more preferably from about 6.5 to about 9.5. The pH is selected to maintain a significant level of binding affinity between the specific binding members. When the label component of the conjugate is an enzyme, however, the pH also must be selected to maintain significant enzyme activity for color development in enzymatic signal production systems. Illustrative buffers include phosphate, carbonate, barbital, diethylamine, tris(hydroxymethyl)aminomethane (Tris), 2-amino-2-methyl-l-propanol and the like. The wicking solution and the test sample can be combined prior to contacting the application pad, or they can be contacted to the application pad sequentially.

Assay reagents, such as the conjugate, can be added directly to the device during the performance of the assay. The preferred embodiment however, involves the incorporation of all necessary assay reagents into the assay device so that only a liquid test sample need be contacted to the device.

The device can be provided in kits for carrying out binding assays of the present invention. For example, a kit can comprise the assay device with its incorporated reagents, and can optionally include a wicking solution and/or test sample pretreatment reagent as described above. Other assay components known to those skilled-in-the-art, such as buffers, stabilizers, detergents, non-specific binding inhibitors, bacteria inhibiting agents and the like can also be present in the assay device and wicking solution.

### EXAMPLES

The following examples are given by way of illustration only and should not be construed as limiting the spirit or scope of the invention as based upon this disclosure. Many variations on the present invention will become obvious to those of ordinary skill-in-the-art.

### Example 1

### Opiate Assay

### a. Conjugate preparation

A conjugate comprising a monoclonal antibody attached to a direct visual label was prepared according to the following procedure. A 0.03% colloidal selenium (5.0 ml; particle size of about 160-170 nm; pH adjusted to 6.5 with 2.0% potassium carbonate) was reacted with a mouse monoclonal anti-morphine antibody (56 µg: raised against 3-8-D-glucuronide morphine Keyhole Limpet Hemocyanin) for ten minutes. The reaction was then blocked by the addition of 10% poly(ethylene glycol) (50 µl; PEG-20,000). After stirring for five minutes, the conjugate was mixed with a diluent (4.0 ml; containing 4.0% sucrose, 2.0% casein, 1.0% Pluronic-104 [BASF, Parsippany, NJ] and 50 mM Tris at pH 7.6). The conjugate was then filtered, using a 0.2 micron filter.

### b. Test strip preparation

Test strips were prepared according to the following procedure. A capture reagent of 3-β-D-glucuronide morphine bovine serum albumin [1.0 mg/ml; in 0.1 M MES (2-[N-morpholino]ethanesulfonic acid) buffer with 20% glycerol and 2.0% sucrose, at pH 5.0] was applied ten millimeters from one edge (i.e., proximal end) of a nitrocellulose sheet (5 micron pore size, Schleichter and Schuell, Keene, NH) in a horizontal line. The capture reagent was capable of competing with analyte in the test sample for binding to the conjugate. A conjugate recovery reagent of goat anti-mouse lgG (0.5 mg/ml; in 0.1 M Tris buffer with 20% glycerol, 2.0% sucrose and 1.0% bovine serum albumin [BSA] at pH 8.0) was applied fifteen millimeters from the same edge of the sheet. The conjugate recovery reagent was capable of binding, and thereby immobilizing, conjugate which passed through the capture site. The sheet was air dried and cut into strips (3 x 60 mm) such that the capture site and conjugate recovery site traversed the width of the strips and were positioned ten millimeters and fifteen millimeters, respectively, from the proximal end of the strips.

### c. Assay protocol

Sample (10 µl) and conjugate (10 µl) were combined in a well to form a test solution. The proximal end of a test strip was placed in the well so that the test solution traveled through the strip to the capture site and conjugate recovery site. When the test mixture reached the far end of the strip, the strip was removed and air dried. The strip was then scanned at 540 nanometers on a reflectance scanner (Camag TLC Scanner II, Multenz, Switzerland).

The lower the amount of analyte in the test sample, the greater the amount of conjugate captured by the analyte-analog at the capture site, and therefore, the greater the signal at the capture site relative to the signal at the conjugate recovery site. As the amount of analyte in the sample increased, the greater was the amount of analyte/conjugate complex formed, and therefore, more conjugate passed through the capture site to be bound at the conjugate recovery site which then showed increased signal. The amount of opiate present in the sample was quantified or detected by observing either the capture site or the conjugate recovery site when the opiate concentration was below 1,000 nanograms/millimeter. In addition, depending upon the analyte concentration, the format of the device enabled the observation of signal at both the capture and recovery sites and thereby provided a highly accurate estimate of analyte concentration. If the opiate concentration was higher than 1,000 nanograms/milliliter, then a much higher signal was detected at the conjugate recovery site than at the capture site. In these cases, the sample was diluted and retested to estimate its concentration. Table 1 shows the results of the assay for various sample concentrations of opiate analyte.

**Table 1**

| Opiate Strip Assay | | | | | | |
|---|---|---|---|---|---|---|
| Relative reflectance | Opiate Sample Concentration (ng/ml) | | | | | |
| units at 540 nm | 0 | 100 | 200 | 350 | 600 | 1000 |
| Capture site | 118 | 82 | 67 | 56 | 54 | 38 |
| Conjugate recovery site | 0 | 13 | 25 | 38 | 42 | 50 |

### Example 2

### Benzodiazepine Assay

### a. Conjugate preparation

A conjugate comprising an antibody attached to a direct visual label was prepared according to the following procedure. A 0.03% colloidal selenium (5.0 ml; particle size of about 160-170 nm; pH adjusted to 7.7 with 2.0% potassium carbonate) was diluted with deionized water (5.0 ml) and was mixed with an affinity purified sheep anti-benzodiazepine antibody (70 µg; raised against 1-carbomethyl-7-chlorobenzodiazepine thyroglobulin) for approximately ten minutes. The reaction was then blocked by the addition of 5.0% BSA (100 µl; in 0.1 M Tris at pH 8.2). After ten minutes, the conjugate was readjusted to pH 7.7 with 2.0% potassium carbonate. The conjugate was then filtered, using a 0.2 µm (micron) filter, and stored at 4°C until used.

### b. Test strip preparation

Test strips were prepared substantially in accordance with the procedure described above in Example 1, with the exception that 1-carbomethyl-7-chlorobenzodiazepine bovine serum albumin (1.0 mg/ml MES buffer) was used to form an analyte-analog capture site, and a donkey anti-sheep lgG antibody (1.0 mg/ml Tris buffer) was used to form the conjugate recovery site.

### c. Assay protocol

Sample (10 µl) conjugate (10 µl) and phosphate buffered saline (10 µl; containing 2.0% casein, 0.1% polyoxyethylene [20] sorbitan monolaurate and 0.05% sodium azide, at pH 7.4) were combined in a well to form a test solution. The proximal end of a strip was placed in the well so that the test solution migrated through the strip to the capture site and conjugate recovery site. When the test mixture reached the far end of the strip, the strip was removed and air dried. The strip was then read as described in Example 1. The differences in the relative signals at the capture site and conjugate recovery site for a variety of benzodiazepine (analyte) concentrations are shown in Table 2. As the analyte concentration was increased, less conjugate was immobilized at the capture site and more conjugate was immobilized at the conjugate recovery site.

**Table 2**

| Benzodiazepine Assay | | | | |
|---|---|---|---|---|
| Relative reflectance | Benzodiazepine Sample Concentration (ng/ml) | | | |
| units at 540 nm | 0 | 200 | 800 | 2400 |
| Capture site | 111 | 86 | 67 | 51 |
| Conjugate recovery site | 50 | 80 | 97 | 103 |

### Example 3

### Theophylline Assay

### a. Conjugate preparation

A conjugate comprising a monoclonal antibody attached to a direct visual label was prepared according to the following procedure. A 0.03% colloidal selenium (5.0 ml; as described above in Example 1; pH adjusted to 6.9 with 2.0% potassium carbonate) was diluted with deionized water (5.0 ml) and was mixed with a monoclonal anti-theophylline antibody (20 µg; mouse antibody raised against 8-theophylline-bovine serum albumin) for approximately ten minutes. The reaction was then blocked by the addition of 5.0% casein (100 µl; in 0.1 M sodium phosphate at pH 7.15). After five minutes, the conjugate was filtered, using a 0.2 µm (micron) filter, and stored at 4°C until used.

### b. Test strip preparation

Test strips were prepared substantially in accordance with the procedure described in Example 1, with the exception that 8-(3'-carbopropyl)-1,3-dimethyl-xanthine-BSA (0.5 mg/ml, in 0.1 M sodium phosphate buffer with 20% glycerol and 2.0% sucrose, at pH 5.0) was used to form the analyte-analog capture site, and a goat anti-mouse lgG antibody (0.3 mg/ml in 0.1 M Tris buffer with 20% glycerol, 2.0% sucrose and 1.0% BSA, at pH 8.0) was used to form the conjugate recovery site. The sites were prepared by hand-dotting lines of reagent on a nitrocellulose sheet with an auto-micropipette.

### c. Assay protocol

The assay protocol was performed substantially in accordance with the protocol described above in Example 2. The differences in the relative signals at the capture site and conjugate recovery site for a variety of theophylline (analyte) concentrations are shown in Table 3. As the analyte concentration was increased, less conjugate was immobilized at the capture site and more conjugate was immobilized at the conjugate recovery site.

**Table 3**

| Theophylline Assay | | | | |
|---|---|---|---|---|
| Relative reflectance | Theophylline Sample Concentration (µg/ml) | | | |
| units at 540 nm | 0 | 2.5 | 10 | 40 |
| Capture site | 138 | 89 | 60 | 46 |
| Conjugate recovery site | 0 | 61 | 76 | 89 |

### Example 4

### Cocaine Assay

For a cocaine assay, a conjugate of labeled anti-cocaine antibody and a test strip were prepared substantially in accordance with the procedures described in Examples 1.a. and 1.b., respectively. The test strip included a capture site containing an analyte-analog capture reagent and a conjugate recovery site containing an anti-antibody, conjugate recovery reagent. The assay protocol was performed substantially in accordance with the protocol described in Example 1.c, using test samples containing from 1.0 to 250 µg/ml of benzoylecgonine. The signal was measured as described in Example 1.

### Example 5

### Rheumatoid Factor Sandwich Assay

### a. Conjugate preparation

A conjugate comprising a rabbit anti-biotin antibody and a direct visual label was prepared according to the following procedure. A 1% gold chloride solution was made with distilled water. Five hundred milliliters of distilled water was then added to ten milliliters of the solution. The resultant solution was heated to a boil, and 1% sodium citrate (8.0 ml) was added. The solution was heated and stirred until the solution became reddish-purple in color. The colloidal gold was then cooled and stored at 4°C until used.

Rabbit anti-biotin antibody (100 µl; 1 mg/ml) was mixed with a 10% BSA solution (100 µl). The colloidal gold (20 ml) was mixed with 150 mM borate buffer (2.0 ml, pH 8.08). The buffered colloidal gold was then added to the anti-biotin antibody/BSA solution and mixed at room temperature for ten minutes. A 10% BSA solution (600 µl) was added to quench the reaction mixture. The mixture was centrifuged at 10,000 rpm for five minutes, the supernatant was removed and the pellet was resuspended 1:1 with 15 mM borate containing 0.2% PEG to form the colloidal gold/rabbit anti-biotin antibody conjugate. The final conjugate concentration was 80 µg/ml. The conjugate was then mixed at a 1:15 ratio with a Tris-buffered saline diluent (TBS; 0.1 mM Tris[hydroxymethl]aminomethane acetate [0.9% saline, pH 7.8] containing 3.0% casein).

### b. Test strip preparation

Test strips were prepared according to the following procedures.

The capture site contained a biotinylated rabbit serum albumin capture reagent (biotin-RSA) which was selected to specifically bind the anti-biotin antibody component of the conjugate. The biotin-RSA was made by preparing a 2% RSA solution in borate buffer saline (pH 8.0). N-Hydroxysuccinimido-Biotin (NHS-biotin; 53.3 mg) was dissolved in dimethyl formamide (1.0 ml), and 100 microliters of the solution was added to the 2% RSA (1.0 ml). The reaction mixture was mixed at room temperature for two hours. The mixture was dialyzed overnight against 0.9% saline, the saline was then changed and the mixture was dialyzed for two more hours. The mixture was then dialyzed for three hours against 200 mM borate (pH 8.4). The resultant biotin-RSA capture reagent was then mixed with a TBS diluent (0.5 mg/ml; containing 0.1% BSA, 1.0% sucrose and 0.003% phenol red).

The conjugate recovery site contained human lgG (HlgG; Sigma, St. Louis, MO) as the conjugate recovery reagent which was selected to indirectly immobilize the conjugate by binding the analyte and any conjugate bound thereto. The conjugate recovery reagent was mixed with the same diluent as was the capture reagent.

A reagent jet was used to dispense the capture reagent and conjugate recovery reagent onto a nitrocellulose sheet. The capture reagent (biotin-RSA, 0.5 mg/ml) was applied in a single pass, ten millimeters from one edge (i.e., proximal end) of the nitrocellulose sheet in a horizontal line (0.03 inches wide). The lgM rheumatoid factor analyte (an anti-lgG antibody) from the test sample competed with the biotin capture reagent for binding to the conjugate. Three conjugate recovery sites of HlgG (5.0 mg/ml) were applied as horizontal lines (0.03 inches wide) at 12.5, 15 and 17.5 millimeter distances from the same edge of the sheet. The first conjugate recovery site was applied in a double pass, and the second and third sites were applied by a single pass The HlgG conjugate recovery reagent was capable of binding the analyte and thereby immobilizing the analyte-bound conjugate which passed through the capture site. The sheet was air dried and cut into strips (4 x 50 mm) such that the capture site and three conjugate recovery sites or bars traversed the width of the strips. The strips were stored with desiccant until used.

### c. Ancillary binding member preparation

A biotinylated HlgG ancillary binding member was prepared according to the following procedure. An HlgG solution (1.0 ml; 1.0 mg/ml in distilled water) was mixed with an NHS-biotin solution (100 µl; 10 mg/ml in distilled water). The reaction mixture was mixed for two hours at 4°C. The reaction was quenched with the addition of TBS (110 µl; 100 mM Tris, 200 mM NaCl, pH 9.0). The mixture was dialyzed overnight against TBS (20 mM Tris, 200 mM NaCl, pH 8.08) at 4°C. The resultant biotin-HlgG ancillary binding member was then mixed at a 1:25 ratio with a TBS diluent (containing 1% PEG) for a final concentration of one microgram/milliliter. The ancillary binding member was used in the assay to increase the amount of biotin present in the binding reaction and thereby increase the amount of conjugate that would become indirectly bound to the analyte, i.e., the analyte binds to the HlgG portion of the ancillary binding member, and a plurality of conjugate structures bind to the biotin component of the ancillary binding member.

The analyte competes with the capture reagent for binding to the conjugate. While the analyte and capture reagent do not bind to the conjugate antibody at the same site, the binding of the analyte to the rabbit anti-biotin antibody portion of the conjugate affects the anti-biotin antibody such that it is unable to bind either the biotin of the ancillary specific binding member or of the capture site.

If no analyte is present in the test sample, then both the conjugate/ancillary binding member complex and free conjugate became immobilized at the capture site via biotin/anti-biotin binding. If analyte is present in the test sample, then there is a conjugate/ancillary binding member complex, a conjugate/ancillary binding member/analyte complex, a conjugate/analyte complex and free conjugate in the reaction mixture. As the analyte concentration increases, the conjugate/ancillary binding member/analyte complex concentration and the conjugate/analyte complex concentration increase, and these complexes pass through the capture site and bind the HlgG of the conjugate recovery sites via the analyte.

### d. Assay protocol

Sample (15 µl), conjugate (7.5 µl) and ancillary binding member (7.5 µl) were combined in a well to form a test solution, and the proximal end of a strip was placed in the well so that the test solution traveled through the strip to reach the capture and recovery sites. After eight minutes, the strip was removed and air dried. The test samples included one rheumatoid factor (RF) negative serum specimen and five RF positive specimens. The strip was then scanned at 540 nanometers on a reflectance scanner, as described in Example 1. The assay results are provided in Table 4.

The results demonstrate that the lower the amount of analyte in the sample, the greater the amount of conjugate captured at the capture site, and therefore, the greater the signal at the capture site relative to the signal at the conjugate recovery sites. Thus, only the capture site had a detectable signal on the strip with which the RF negative sample was tested. As the amount of analyte in the sample was increased, the greater the amount of conjugate/ancillary binding member/analyte complex and conjugate/analyte complex formed, and therefore, more bound conjugate passed through the capture site to be immobilized at the conjugate recovery sites which showed an increase in detectable signal. As illustrated in Table 4, the greater the amount of analyte present in the sample the greater the number of conjugate recovery sites having a detectable signal and/or the greater the signal at each site.

**Table 4**

| Rheumatoid Factor Assay | | | | | | |
|---|---|---|---|---|---|---|
| Relative reflectance | RF Sample Titer | | | | | |
| units at 540 nm | 0 | 20 | 160 | 1280 | 10240 | 81920 |
| Capture site | 109 | 100 | 94.33 | 78.3 | 54.6 | 52.1 |
| Conjugate recovery sites | | | | | | |
| 1 | 0 | 5.9 | 33.0 | 48.7 | 72.9 | 45.4 |
| 2 | 0 | 0 | 0 | 8.2 | 10.7 | 14.7 |
| 3 | 0 | 0 | 0 | 0 | 6.9 | 8.5 |

The dual readout system of the present invention as well as the optional polyvalent conjugates are applicable to various types of solid phase binding assays. It will be appreciated, however, that one skilled-in-the-art can conceive of other solid phase materials and of analytes other than antigens or antibodies, to which the present invention can be applied. The embodiments described and the alternative embodiments presented are intended as examples rather than as limitations. Thus, the detailed description of the invention is not intended to limit the invention to the particular embodiments disclosed, but it is intended to encompass all equivalents and subject matter within the scope of the invention as described above and as set forth in the following claims.

## Claims

1. A method for determining the amount of an analyte of interest in a test sample, comprising the steps of:
a) contacting the test sample to a solid phase through which the analyte and a conjugate are capable of migrating, said conjugate comprising a specific binding member attached to a label, wherein said solid phase is incorporated with at least two defined detection sites, in sequential fluid-flow contact, for the immobilization and comparative display of a member selected from said conjugate and complexes thereof,
said detection sites comprising
i) a capture site comprising an immobilized capture reagent capable of competing with the analyte for binding to said conjugate, and
ii) ) a conjugate recovery site comprising an immobilized conjugate recovery reagent, different from said capture reagent, and capable of binding a member selected from said conjugate and complexes thereof, wherein said conjugate recovery site receives and binds said conjugate or conjugate complexes which migrate through said capture site;
b) allowing the test sample to migrate through said detection sites; and
c ) detecting immobilized conjugate at both said capture site and said conjugate recovery site by instrumental means to quantitatively determine the amount of the analyte of interest.

2. A method for determining the amount of an analyte of interest in a test sample, comprising the steps of:
a) contacting the test sample to a solid phase through which the analyte and a conjugate are capable of migrating, said conjugate comprising a specific binding member attached to a label, wherein said solid phase is incorporated with at least two defined detection sites, in sequential fluid-flow contact, for the immobilization and comparative display of a member selected from said conjugate and complexes thereof,
said detection sites comprising
i ) a capture site comprising an immobilized capture reagent to which the analyte and conjugate competitively bind, and
ii) a conjugate recovery site comprising an immobilized conjugate recovery reagent, different from
said capture reagent, and capable of binding a member selected from said label, an epitope peculiar to said conjugate and an ancillary specific binding member, wherein said conjugate recovery site receives and binds said conjugate or conjugate complexes which migrate through said capture site;
b) allowing the test sample to migrate through said detection sites; and
c) detecting immobilized conjugate at both said capture site and said conjugate recovery site by instrumental means to quantitatively determine the amount of the analyte of interest.

3. A method for determining the amount of an analyte of interest in a test sample, comprising the steps of:
a) contacting the test sample to a solid phase device through which the analyte and a conjugate are capable of migrating, wherein said solid phase is incorporated with at least two defined detection sites, in sequential fluid-flow contact, for the immobilization and comparative display of a member selected from said conjugate and complexes thereof,
said detection sites comprising
i ) a capture site comprising an immobilized capture reagent capable of competing with the analyte for binding to said conjugate, and
ii) a conjugate recovery site comprising an immobilized conjugate recovery reagent, different from said capture reagent, and capable of binding a member selected from the analyte, analyte complexes and analyte-specific ancillary binding members,
wherein said conjugate recovery site receives and binds said conjugate and conjugate complexes which migrate through said capture site, and
wherein said conjugate comprises a label attached to a specific binding member having a binding site specific for the analyte and a binding site specific for said capture reagent, and wherein the binding of the analyte and said conjugate inhibits the binding of said capture reagent and said conjugate, and wherein the binding of said conjugate and said capture reagent inhibits the binding of the analyte and said conjugate;
b) allowing the test sample to travel through said solid phase; and
c) detecting immobilized conjugate at both said capture site and said conjugate recovery site by instrumental means to quantitatively determine the amount of an analyte of interest in a test sample.

## Patentansprüche

1. Verfahren zur Bestimmung der Menge eines interessierenden Analyten in einer Testprobe, das folgende Schritte umfaßt:
a) In-Kontakt-Bringen der Testprobe mit einer festen Phase, durch die der Analyt und ein Konjugat zu wandern vermögen, wobei das Konjugat ein spezifisch bindendes Glied umfaßt, das an eine Markierung gebunden ist, wobei in der festen Phase wenigstens zwei definierte Nachweisstellen eingebaut sind, die sich in aufeinander abfolgendem Kontakt mit dem Flüssigkeitsfluß befinden, und die der Immobilisierung und der vergleichenden Anzeige eines Gliedes dienen, daß aus dem Konjugat und dessen Komplexen gewählt ist, wobei die Nachweisstellen folgendes umfassen:
i) eine Einfangstelle, die ein immobilisiertes Einfangreagenz umfaßt, daß zur Konkurrenz mit dem Analyten zur Bindung an das Konjugat befähigt ist, und
ii) eine Konjugatgewinnungsstelle, die ein immobilisiertes Konjugatgewinnungsreagenz umfaßt, das von dem Einfangreagenz verschieden ist, und das zur Bindung eines Gliedes befähigt ist, das aus dem Konjugat und dessen Komplexen gewählt ist, wobei die Konjugatgewinnungsstelle das Konjugat oder die Konjugatkomplexe aufnimmt und bindet, das/die durch die Einfangstelle hindurchwandert/n;
b) der Testprobe zu erlauben, durch die Nachweisstellen hindurchzuwandern; und
c) das Nachweisen des immobilisierte Konjugates sowohl an der Einfangstelle als auch an der Konjugatgewinnungsstelle durch instrumentelle Mittel, um die Menge des interessierenden Analyten quantitativ zu bestimmen.

2. Verfahren zur Bestimmung der Menge eines interessierenden Analyten in einer Testprobe, das folgende Schritte umfaßt:
a) In-Kontakt-Bringen der Testprobe mit einer festen Phase, durch die der Analyt und ein Konjugat hindurchzuwandern vermögen, wobei das Konjugat ein spezifisch bindendes Glied umfaßt, das an eine Markierung gebunden ist, wobei in der feste Phase wenigstens zwei definierte Nachweisstellen eingebaut sind, die sich in aufeinander abfolgendem Kontakt mit dem Flüssigkeitsfluß befinden, und die der Immobilisierung und der vergleichenden Anzeige eines Gliedes dienen, das aus dem Konjugat und dessen Komplexen gewählt ist, wobei die Nachweisstellen folgendes umfassen:
i) eine Einfangstelle, die ein immobilisiertes Einfangreagenz umfaßt, an das der Analyt und das Konjugat konkurrierend binden, und
ii) eine Konjugatgewinnungsstelle, die ein immobilisiertes Konjugatgewinnungsreagenz umfaßt, das von dem Einfangreagenz verschieden ist, und das zur Bindung eines Gliedes befähigt ist, das aus der Markierung, einem Epitop, das für das Konjugat eigentümlich ist, und einem spezifisch bindenden Hilfsglied gewählt ist, wobei die Konjugatgewinnungsstelle das Konjugat oder die Konjugatkomplexe aufnimmt und bindet, die durch die Einfangstelle hindurchwandern;
b) der Testprobe zu erlauben, durch die Nachweisstellen hindurchzuwandern; und
c) das Nachweisen des immobilisierten Konjugates sowohl an der Einfangsstelle als auch an der Konjugatgewinnungsstelle mittels instrumenteller Mittel, um die Menge des interessierenden Analyten quantitativ zu bestimmen.

3. Verfahren zur Bestimmung der Menge eines interessieremden Analyten in einer Testprobe, das die folgenden Schritte umfaßt:
a) In-Kontakt-Bringen der Testprobe mit einer Festphasenvorrichtung, durch die der Analyt und ein Konjugat hindurchzuwandern vermögen, wobei in der festen Phase wenigstens zwei definierte Nachweisstellen eingebaut sind, die sich in aufeinander abfolgendem Kontakt mit dem Flüssigkeitsfluß befinden, zur Immobilisierung und zur vergleichenden Anzeige eines Gliedes, aas aus dem Komjugat und dessen Komplexen gewählt ist, wobei die Nachweisstellen folgendes umfassen:
i) eine Einfangstelle, die ein immobilisiertes Einfangreagenz umfaßt, daß zur Konkurrenz mit dem Analyten zur Bindung an das Konjugat befähigt ist, und
ii) eine Konjugatgewinnungsstelle, die ein immobilisiertes Konjugatgewinnungsreagenz umfaßt, das von dem Einfangreagenz verschieden ist, und das zur Bindung eines Gliedes befähigt ist, das aus dem Analyten, den Analytkomplexen und analyt-spezifischen Hilfsbindungsgliedern gewählt ist,
wobei die Konjugatgewinnungsstelle das Konjugat und die Konjugatkomplexe, die durch die Einfangsstelle wandern, aufnimmt und bindet, und
wobei das Konjugat eine Markierung umfaßt, die an ein spezifisch bindendes Glied gebunden ist, das eine Bindungsstelle aufweist, die für den Analyten spezifisch ist, und eine Bindungsstelle, die für das Einfangreagenz spezifisch ist, und wobei die Bindung des Analyten und des Konjugates die Bindung des Einfangreagenzes und des Konjugates hemmt, und wobei die Bindung des Konjugates und des Einfangreagenzes und die Bindung des Analyten und des Konjugates hemmt;
b) der Testprobe zu erlauben, durch die feste Phase hindurchzuwandern; und
c) das Nachweisen des Immobilisierten Konjugates sowohl an der Einfangsstelle als auch an der Konjugatgewinnungsstelle durch instrumentelle Mittel, um die Menge eines Interessierenden Analyten in einer Testprobe quantitativ zu bestimmen.

## Revendications

1. Procédé de détermination de la quantité d'un analyte intéressant dans un échantillon à doser, comprenant les étapes consistant à :
a) mettre l'échantillon à doser en contact avec une phase solide à travers laquelle l'analyte et un conjugué sont capables de migrer, ledit conjugué comprenant un élément à liaison spécifique combiné à un marqueur, ladite phase solide comportant au moins deux sites de détection définis, en contact successif par écoulement de liquide, en vue de l'immobilisation et de l'affichage comparatif d'un élément sélectionné parmi ledit conjugué et ses complexes, lesdits sites de détection comprenant :
i) un site de piégeage comprenant un réactif de piégeage immobilisé, capable d'entrer en compétition avec l'analyte pour la fixation audit conjugué et
ii) un site de récupération de conjugué comprenant un réactif de récupération de conjugué immobilisé, différent dudit réactif de piégeage, et capable de fixer un élément sélectionné parmi ledit conjugué et ses complexes, ledit site de récupération de conjugué recevant et fixant ledit conjugué ou lesdits complexes du conjugué qui migrent à travers ledit site de piégeage ;
b) laisser migrer l'échantillon à doser à travers lesdits sites de détection et
c) détecter le conjugué immobilisé audit site de piégeage et audit site de récupération de conjugué par des moyens instrumentaux pour déterminer quantitativement la quantité d'analyte intéressant.

2. Procédé de détermination de la quantité d'un analyte intéressant dans un échantillon à doser, comprenant les étapes consistant à :
a) mettre l'échantillon à doser en contact avec une phase solide à travers laquelle l'analyte et un conjugué sont capables de migrer, ledit conjugué comprenant un élément à liaison spécifique combiné à un marqueur, ladite phase solide comportant au moins deux sites de détection définis, en contact successif par écoulement de liquide, en vue de l'immobilisation et de l'affichage comparatif d'un élément sélectionné parmi ledit conjugué et ses complexes,
lesdits sites de détection comprenant
i) un site de piégeage comprenant un réactif de piégeage immobilisé auquel l'analyte et le conjugué se fixent compétitivement et
ii) un site de récupération de conjugué comprenant un réactif de récupération de conjugué immobilisé, différent dudit réactif de piégeage, et capable de fixer un élément sélectionné parmi ledit conjugué, un épitope particulier audit conjugué et un élément à liaison spécifique auxiliaire, ledit site de récupération de conjugué recevant et fixant ledit conjugué ou lesdits complexes du conjugué qui migrent à travers ledit site de piégeage ;
b) laisser l'échantillon à doser migrer à travers ledits sites de détection et
c) détecter le conjugué immobilisé audit site de piégeage et audit site de récupération de conjugué par des moyens instrumentaux pour déterminer quantitativement la quantité d'analyte intéressant.

3. Procédé de détermination de la quantité d'un analyte intéressant dans un échantillon à doser, comprenant les étapes consistant à :
a) mettre l'échantillon à doser en contact avec un dispositif à phase solide à travers lequel l'analyte et un conjugué sont capables de migrer, ladite phase solide comportant au moins deux sites de détection définis, en contact successif par écoulement de liquide, en vue de l'immobilisation et de l'affichage comparatif d'un élément sélectionné parmi ledit conjugué et ses complexes,
lesdits sites de détection comprenant
i) un site de piégeage comprenant un réactif de piégeage immobilisé, capable d'entrer en compétition avec l'analyte pour la fixation audit conjugué et
ii) un site de récupération de conjugué comprenant un réactif de récupération de conjugué immobilisé, différent dudit réactif de piégeage et capable de fixer un élément sélectionné parmi l'analyte, des complexes de l'analyte et des éléments de liaison auxiliaires spécifiques de l'analyte,
ledit site de récupération de conjugué recevant et fixant ledit conjugué et lesdits complexes du conjugué qui migrent à travers ledit site de piégeage et
ledit conjugué comprenant un marqueur combiné à un élément à liaison spécifique ayant un site de liaison spécifique pour l'analyte et un site de liaison spécifique sous ledit réactif de piégeage, la fixation de l'analyte et dudit conjugué inhibant la fixation dudit réactif de piégeage et dudit conjugué et la fixation dudit conjugué et dudit réactif de piégeage inhibant la fixation de l'analyte et dudit conjugué ;
b) laisser l'échantillon à doser se déplacer à travers ladite phase solide et
c) détecter le conjugué immobilisé audit site de piégeage et audit site de récupération de conjugué par des moyens instrumentaux pour déterminer quantitativement la quantité d'analyte intéressant dans un échantillon à doser.
